# EUROPEAN PATENT SPECIFICATION

(11) **EP 4 121 551 B1**
(45) Date of publication and mention of the grant of the patent: **08.07.2026**
(21) Application number: 21714838.6
(22) Date of filing: 19.03.2021
(51) Int. Cl.: C12Q 1/28, G01N 33/569, G01N 33/68

(54) **TRIAGING METHOD USING CELL FREE NUCLEOSOME LEVELS**
TRIAGING-VERFAHREN UNTER VERWENDUNG ZELLFREIER NUKLEOSOMSPIEGEL
PROCÉDÉ DE TRIAGE UTILISANT DES NIVEAUX DE NUCLÉOSOMES ACELLULAIRES

(30) Priority: 20.03.2020 GB 202004100; 07.04.2020 CN 202010265531; 06.05.2020 GB 202006723; 07.07.2020 GB 202010446; 10.09.2020 GB 202014263; 16.10.2020 GB 202016403; 30.11.2020 GB 202018835; 20.01.2021 GB 202100769
(43) Date of publication of application: 25.01.2023
(60) Divisional application: 23169604.8
(73) Proprietor: Belgian Volition SRL, 5032 Isnes (BE)
(72) Inventor: ECCLESTON, Mark Edward, 5032 Isnes (BE); MICALLEF, Jacob Vincent, 5032 Isnes (BE); TERRELL, Jason Bradley, 5032 Isnes (BE)
(74) Representative: Sagittarius IP
(86) International application number: PCT/EP2021/057096
(87) International publication number: WO 2021/186037

(56) References cited:
- WO-A1-2018/083308
- WO-A1-2020/055597
- US-A1- 2014 057 829
- NOMURA KOZO ET AL: "Citrullinated Histone H3: Early Biomarker of Neutrophil Extracellular Traps in Septic Liver Damage", JOURNAL OF SURGICAL RESEARCH, ACADEMIC PRESS INC., SAN DIEGO, CA, US, vol. 234, 10 October 2018 (2018-10-10), pages 132 - 138, XP085557744, ISSN: 0022-4804, DOI: 10.1016/J.JSS.2018.08.014
- DENG QIUFANG ET AL: "Citrullinated Histone H3 as a Therapeutic Target for Endotoxic Shock in Mice", FRONTIERS IN IMMUNOLOGY, vol. 10, 9 January 2020 (2020-01-09), XP93065534, DOI: 10.3389/fimmu.2019.02957
- Meeting abstract Intenisve case medicine experimental 2024 12:87 Sue et al. "Measurement of circulating necleosomes in septic shock"
- EBRAHIMI FAHIM ET AL: "Markers of neutrophil extracellular traps predict adverse outcome in community-acquired pneumonia: secondary analysis of a randomised controlled trial", EUROPEAN RESPIRATORY JOURNAL, vol. 51, no. 4, 8 March 2018 (2018-03-08), GB, pages 1701389, XP055794603, ISSN: 0903-1936, Retrieved from the Internet <URL:https://erj.ersjournals.com/content/erj/51/4/1701389.full.pdf> DOI: 10.1183/13993003.01389-2017
- TOMOYA HIROSE ET AL: "Presence of Neutrophil Extracellular Traps and Citrullinated Histone H3 in the Bloodstream of Critically Ill Patients", PLOS ONE, vol. 9, no. 11, 13 November 2014 (2014-11-13), pages e111755, XP055242707, DOI: 10.1371/journal.pone.0111755
- LEE K. H. ET AL: "Quantification of NETs-associated markers by flow cytometry and serum assays in patients with thrombosis and sepsis", INTERNATIONAL JOURNAL OF LABORATORY HEMATOLOGY, vol. 40, no. 4, 9 March 2018 (2018-03-09), GB; US, pages 392 - 399, XP055797734, ISSN: 1751-5521, Retrieved from the Internet <URL:https://api.wiley.com/onlinelibrary/tdm/v1/articles/10.1111%2Fijlh.12800> DOI: 10.1111/ijlh.12800
- THÅLIN CHARLOTTE ET AL: "Validation of an enzyme-linked immunosorbent assay for the quantification of citrullinated histone H3 as a marker for neutrophil extracellular traps in human plasma", IMMUNOLOGIC RESEARCH, HUMANA PRESS, INC, US, vol. 65, no. 3, 4 February 2017 (2017-02-04), pages 706 - 712, XP036242246, ISSN: 0257-277X, [retrieved on 20170204], DOI: 10.1007/S12026-017-8905-3
- ASHAR HARSHINI K ET AL: "The Role of Extracellular Histones in Influenza Virus Pathogenesis", THE AMERICAN JOURNAL OF PATHOLOGY, vol. 188, no. 1, 1 January 2018 (2018-01-01), XP055797944, Retrieved from the Internet <URL:https://www.ncbi.nlm.nih.gov/pmc/articles/PMC5745522/pdf/main.pdf>
- KAUFMAN TOMÁS ET AL: "Nucleosomes and neutrophil extracellular traps in septic and burn patients", CLINICAL IMMUNOLOGY, vol. 183, 30 August 2017 (2017-08-30), pages 254 - 262, XP085256965, ISSN: 1521-6616, DOI: 10.1016/J.CLIM.2017.08.014
- ZUO YU ET AL: "Neutrophil extracellular traps in COVID-19", JCI INSIGHT, vol. 5, 4 June 2020 (2020-06-04), XP055797718, Retrieved from the Internet <URL:https://insight.jci.org/articles/view/138999/version/2/pdf/render.pdf> DOI: 10.1172/jci.insight.138999
- QIXING CHEN ET AL: "Circulating nucleosomes as a predictor of sepsis and organ dysfunction in critically ill patients", INTERNATIONAL JOURNAL OF INFECTIOUS DISEASES, INTERNATIONAL SOCIETY FOR INFECTIOUS DISEASES, HAMILTON, CA, vol. 16, no. 7, 9 March 2012 (2012-03-09), pages e558 - e564, XP028493944, ISSN: 1201-9712, [retrieved on 20120501], DOI: 10.1016/J.IJID.2012.03.007
- YOKOYAMA YAYOI ET AL: "Circulating histone H3 levels in septic patients are associated with coagulopathy, multiple organ failure, and death: a single-center observational study", THROMBOSIS JOURNAL, vol. 17, no. 1, 1 December 2019 (2019-12-01), XP055806793, Retrieved from the Internet <URL:https://www.ncbi.nlm.nih.gov/pmc/articles/PMC6330748/pdf/12959_2018_Article_190.pdf> DOI: 10.1186/s12959-018-0190-4
- ITO TAKASHI ET AL: "Serum histone H3 levels and plateletcounts are potential markers forcoagulopathy with high risk of death inseptic patients: a single-centerobservational study", JOURNAL OF INTENSIVE CARE, vol. 7, no. 1, 1 December 2019 (2019-12-01), XP055797657, Retrieved from the Internet <URL:https://jintensivecare.biomedcentral.com/track/pdf/10.1186/s40560-019-0420-2.pdf> DOI: 10.1186/s40560-019-0420-2
- KUMAR SANNI ET AL: "Quantification of NETs formation in neutrophil and its correlation with the severity of sepsis and organ dysfunction", CLINICA CHIMICA ACTA, vol. 495, 1 August 2019 (2019-08-01), AMSTERDAM, NL, pages 606 - 610, XP055806795, ISSN: 0009-8981, Retrieved from the Internet <URL:https://www.sciencedirect.com/science/article/pii/S0009898119318996/pdfft?md5=b5449523693d150c0c5360cb4c793dba&pid=1-s2.0-S0009898119318996-main.pdf> DOI: 10.1016/j.cca.2019.06.008
- ZEERLEDER SACHA ET AL: "Elevated nucleosome levels in systemic inflammation and sepsis", CRITICAL CARE MEDICINE, LIPPINCOTT WILLIAMS & WILKINS, US, vol. 31, no. 7, 1 January 2003 (2003-01-01), pages 1947 - 1951, XP008158587, ISSN: 0090-3493, DOI: 10.1097/01.CCM.0000074719.40109.95
- DELABRANCHE XAVIER ET AL: "Evidence of Netosis in Septic Shock-Induced Disseminated Intravascular Coagulation", SHOCK, vol. 47, no. 3, 1 March 2017 (2017-03-01), US, pages 313 - 317, XP055797927, ISSN: 1073-2322, DOI: 10.1097/SHK.0000000000000719
- SAUKKONEN KATRI ET AL: "Cell-Free Plasma DNA as a Predictor of Outcome in Severe Sepsis and Septic Shock", CLINICAL CHEMISTRY, vol. 54, no. 6, 1 June 2008 (2008-06-01), US, pages 1000 - 1007, XP055806792, ISSN: 0009-9147, Retrieved from the Internet <URL:https://academic.oup.com/clinchem/article-pdf/54/6/1000/32671963/clinchem1000.pdf> DOI: 10.1373/clinchem.2007.101030
- JUN XU ET AL: "Extracellular histones are major mediators of death in sepsis", NATURE MEDICINE, vol. 15, no. 11, 1 November 2009 (2009-11-01), pages 1318 - 1321, XP055082137, ISSN: 1078-8956, DOI: 10.1038/nm.2053
- ZHU LIULUAN ET AL: "High Level of Neutrophil Extracellular Traps Correlates With Poor Prognosis of Severe Influenza A Infection", JOURNAL OF INFECTIOUS DISEASES, vol. 217, no. 3, 9 January 2018 (2018-01-09), US, pages 428 - 437, XP055794600, ISSN: 0022-1899, DOI: 10.1093/infdis/jix475
- LV XIN ET AL: "Extracellular histones are clinically relevant mediators in the pathogenesis of acute respiratory distress syndrome", RESPIRATORY RESEARCH, vol. 18, no. 1, 1 December 2017 (2017-12-01), XP055797959, Retrieved from the Internet <URL:https://respiratory-research.biomedcentral.com/track/pdf/10.1186/s12931-017-0651-5.pdf> DOI: 10.1186/s12931-017-0651-5
- YEHYA NADIR ET AL: "Circulating nucleosomes are associated with mortality in pediatric acute respiratory distress syndrome.", THE AMERICAN JOURNAL OF PHYSIOLOGY, 1 June 2016 (2016-06-01), pages L1177 - 1184, XP055806922, Retrieved from the Internet <URL:https://journals.physiology.org/doi/pdf/10.1152/ajplung.00067.2016> [retrieved on 20210525], DOI: 10.1152/ajplung.00067.2016.
- XU ZHIHENG ET AL: "Sepsis and ARDS: The Dark Side of Histones", MEDIATORS OF INFLAMMATION., vol. 2015, 1 January 2015 (2015-01-01), GB, pages 1 - 9, XP055806926, ISSN: 0962-9351, Retrieved from the Internet <URL:https://www.ncbi.nlm.nih.gov/pmc/articles/PMC4644547/pdf/MI2015-205054.pdf> DOI: 10.1155/2015/205054
- GRAILER JAMISON J ET AL: "Lung inflammation and damage induced by extracellular histones", INFLAMMATION AND CELL SIGNALING, 1 January 2014 (2014-01-01), XP055806942, Retrieved from the Internet <URL:https://www.ncbi.nlm.nih.gov/pmc/articles/PMC4457458/pdf/nihms688812.pdf> DOI: 10.14800/ics.131

## Description

### FIELD OF THE INVENTION

The present invention relates to the use of cell free nucleosomes comprising histone H3.1 as biomarkers in body fluid samples for patients with sepsis or septic shock, particularly for identifying patients at high risk of developing a NETosis associated adverse reaction to sepsis or septic shock.

### BACKGROUND OF THE INVENTION

Influenza spreads around the world in yearly outbreaks, resulting in about three to five million cases of severe illness and about 290,000 to 650,000 deaths. More recently, the emergence and rapid progression of a new infection, COVID-19, has escalated to pandemic status. Some infections lead to acute respiratory syndrome (ARS), acute respiratory distress syndrome (ARDS), or to severe acute respiratory syndrome (SARS) which are potentially lethal disease progressions requiring medical treatment. Infection outbreaks and pandemics place severe strain on international health care services, therefore methods of triaging patients to identify those who are most likely to require hospital intervention are critical in helping health care providers to prioritise patients, save lives and manage higher demands on medical services more effectively.

COVID-19, influenza and other infections have the potential to progress to the involvement of NETosis related complications which may be severe and can be lethal. Such complications include sepsis, a life-threatening organ dysfunction that can occur as a complication to infection. Methods to treat inappropriate NETosis, to identify individuals at high risk of NETosis related complications, to monitor the progress of such complications in need of such treatment, to monitor the efficacy of treatments and to monitor the progress of such disease are currently lacking.

Holdenrieder et al., Int. J. Cancer (2001) 95: 114-120 previously described detecting the level of nucleosomes in serum samples of patients with benign and malignant diseases. The epigenetic composition of circulating cell free nucleosomes in terms of their histone modification, histone variant, DNA modification and adduct content have also been investigated as blood based biomarkers in cancer, see WO 2005/019826, WO 2013/030577, WO 2013/030579 and WO 2013/084002. Yokoyama et al. Thrombosis Journal (2019) 17:1 discloses how circulating histone H3 levels are associated with coagulopathy, multiple organ failure, and death in patients requiring intensive care because of infectious diseases. Ito et al. Journal of Intensive Care (2019) 7:63 discloses how serum histone H3 levels and platelet counts are potential markers for determining coagulopathy with high risk of death in septic patients.

There remains a need in the art to provide effective treatments for NETosis related conditions as well as for simple, cost-effective methods to identify and prioritize individuals likely to develop NETosis related complications with poor prognosis upon infection and to monitor treatment and progress of disease.

### BRIEF DESCRIPTION OF FIGURES

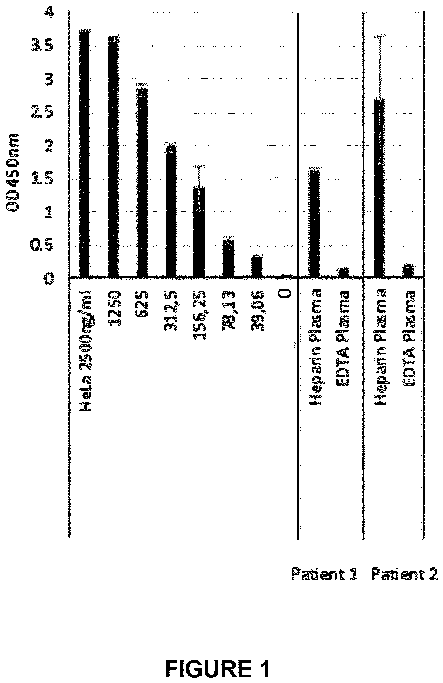

**Figure 1****.** The results of an immunoassay for neutrophil extracellular trap (NET) derived nucleosomes in EDTA plasma and heparin plasma samples taken from 2 healthy volunteers. The EDTA samples contain low levels of NET derived nucleosome material. In contrast, heparin induces NET formation and the heparin plasma samples contain high levels of induced NET derived nucleosomes.

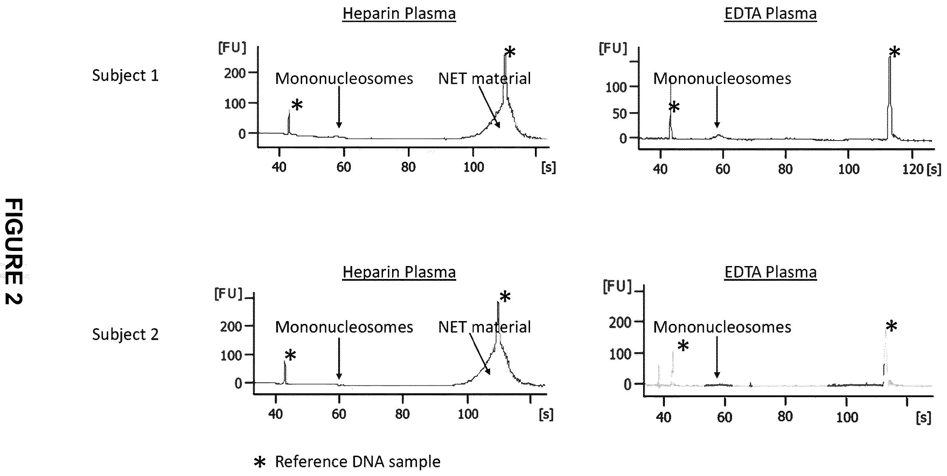

**Figure 2****.** Bioanalyzer electrophoresis results for NET derived nucleosomes in EDTA plasma and heparin plasma samples taken from 2 healthy volunteers. The EDTA samples contain low levels of both mononucleosomes and NET derived nucleosome material. In contrast, heparin induces NET formation and the heparin plasma samples contain low levels of mononucleosomes (peak at approximately 60 seconds) but high levels of induced NET derived nucleosomes (wide peak at approximately 110 seconds). The narrow peaks at approximately 43 seconds and approximately 110 seconds represent DNA samples added for reference purposes.

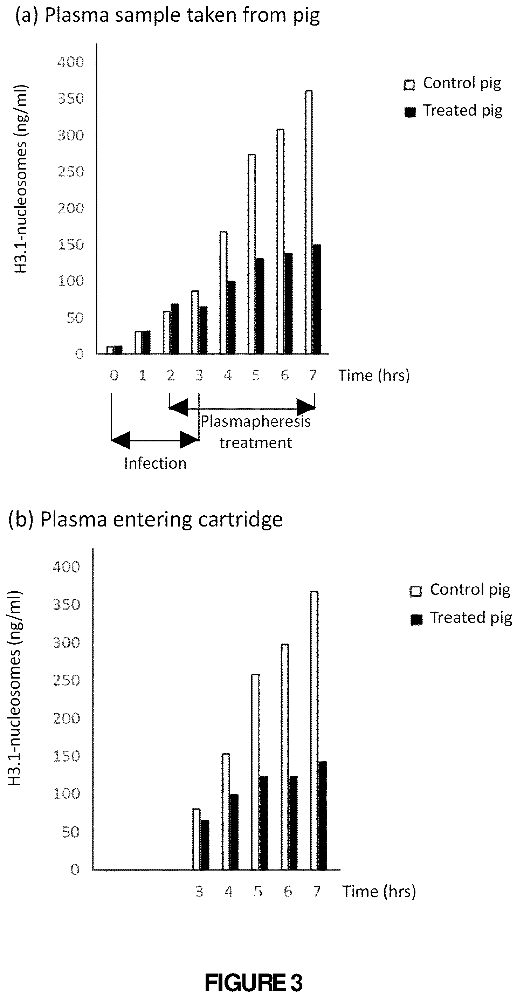

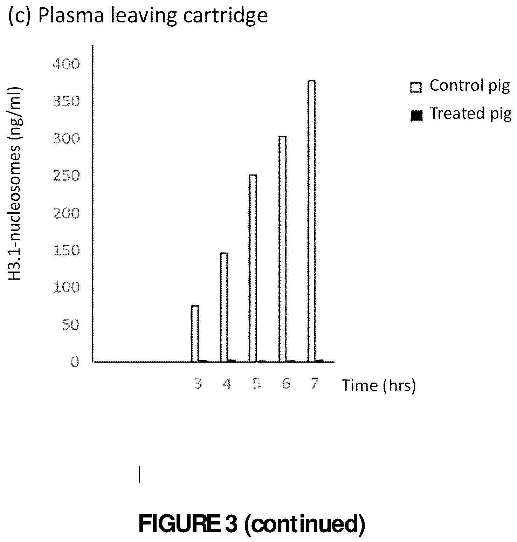

**Figure 3****.** Results from the experiment described in Example 3 showing the mean levels of nucleosomes containing histone isoform H3.1 measured in 16 pigs induced with sepsis placed on plasmapheresis. In 9 pigs, plasma was passed through a cartridge containing NET binders (treated, closed bars) and in 7 pigs, plasma was passed through a control cartridge which did not contain a NETs binder (control, open bars).

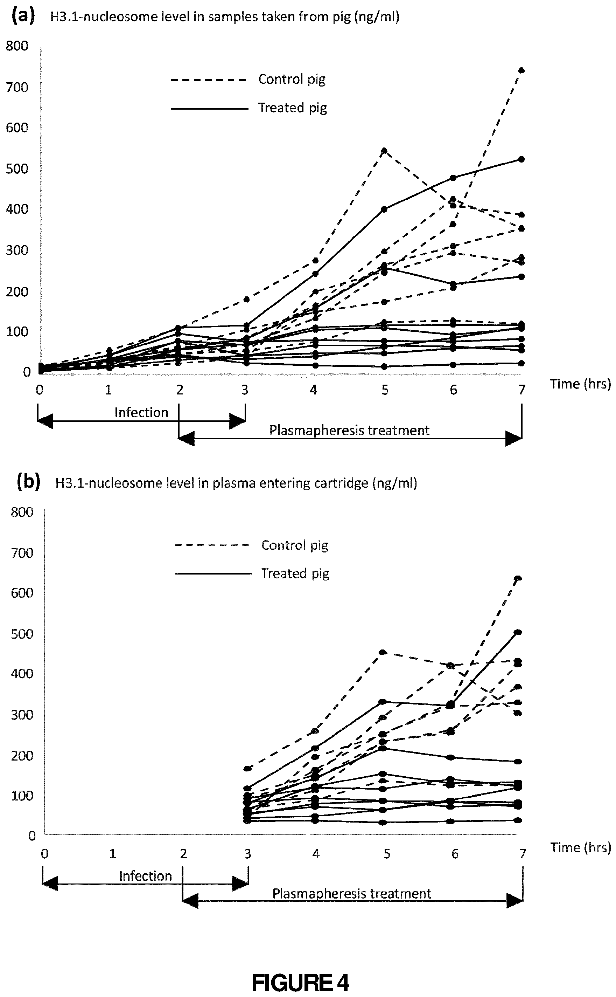

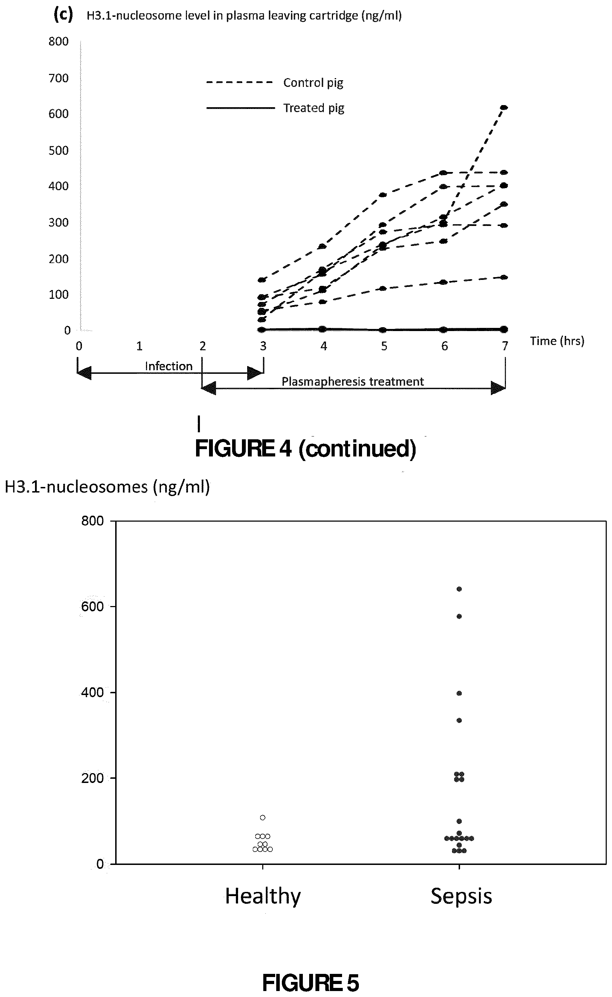

**Figure 4****.** Results from the experiment described in Example 3 and shown in Figure 3, but for levels in individual test subjects.
**Figure 5****.** H3.1-nucleosome levels measured in human subjects diagnosed with sepsis and healthy human subjects.

### SUMMARY OF THE INVENTION

According to a first aspect, there is provided a method of monitoring the progress of sepsis or septic shock in a subject, comprising:
(i) contacting a body fluid sample obtained from the subject with a binding agent to detect or measure the level of cell free nucleosomes comprising histone H3.1;
(ii) repeating step (i) on one or more occasions; and
(iii) using any changes in the level of cell free nucleosomes comprising histone H3.1 to monitor the progression of sepsis or septic shock in the subject,
wherein the method is a two-site immunoassay method that employs an immobilized anti-histone H3.1 antibody directed to bind to a core histone epitope of histone H3.1 within amino acids 29-35 of the histone H3.1 protein together with a labelled anti-nucleosome antibody directed to bind to a conformational epitope present in intact nucleosomes.

According to a further aspect, there is provided a method of assigning a risk of an adverse outcome to a subject suffering from sepsis or septic shock, comprising:
(i) contacting a body fluid sample obtained from the subject with a binding agent to detect or measure the level of cell free nucleosomes comprising histone H3.1; and
(ii) using the level of cell free nucleosomes comprising histone H3.1 detected to assign the likelihood of an adverse outcome to said subject,

wherein a subject identified with a high likelihood of an adverse outcome is assigned for medical intervention, and
wherein the method is a two-site immunoassay method that employs an immobilized anti-histone H3.1 antibody directed to bind to a core histone epitope of histone H3.1 within amino acids 29-35 of the histone H3.1 protein together with a labelled anti-nucleosome antibody directed to bind to a conformational epitope present in intact nucleosomes.

### DETAILED DESCRIPTION

Nucleosomes are released into the circulation on fragmentation of chromatin on cell death. Many infections, such as viral infections, initiate cell death through a variety of mechanisms (cell binding and entry, endosomal TLR3 activation and gene expression) thereby increasing the number of circulating nucleosomes in the blood (Danthi et al., Annu. Rev. Virol. (2016) 3: 533-53). In addition, infections can induce NETosis whereby post-translational histone modifications, such as acetylation or hypercitrullination of histones H3 and H4 (Wang Y et al., J. Cell Biol. (2009) 184(2): 205-213), promote decondensation of chromatin which is released into circulation together as a first line response to infection. However, extracellular nucleosomes and neutrophil extracellular traps (NETs) can cause severe complications if not cleared rapidly. For example, nucleosome binding to the glomerular membrane is associated with kidney damage in lupus (Kalaaji et al., Kidney Int. (2007) 71(7): 665-672), whilst NETs have been shown to intensify pulmonary injury during viral pneumonia (Ashar et al., Am. J. Pathol. (2018) 188(1): 135-148). Indeed, host directed NET toxicity is associated with respiratory distress, occlusion of narrow airways, endothelial and epithelial cell damage, inflammatory response and thrombus formation and other pathologies (Marcos et al., Nat. Med. (2010) 16: 1018-23; Hoeksema et al., Future Microbiol. (2016) 11: 441-53).

Most subjects infected with influenza or coronavirus experience mild illness. However, some population subgroups, including elderly persons aged over 60 years and persons with an underlying medical condition such as diabetes, chronic lung conditions and particularly chronic cardiac conditions, are at risk of severe effects including ARS, SARS, pneumonia and death. The exact mechanism by which influenza or coronavirus infection leads to complications including pneumonia is not clear, but it is thought to be caused by a hyperimmune reaction to the viral infection in which excessive NETs contribute to acute injury of the lung leading to pneumonia and, in the worst cases, death.

The present invention utilises elevated levels of cell free nucleosomes comprising histone H3.1, to predict severity of disease and outcome in sepsis or septic shock.

Therefore, according to one aspect, there is provided a method of assigning a risk of an adverse outcome to a subject suffering from sepsis or septic shock, comprising:
(i) contacting a body fluid sample obtained from the subject with a binding agent to detect or measure the level of cell free nucleosomes comprising histone H3.1; and
(ii) using the level of cell free nucleosomes comprising histone H3.1 detected to assign the likelihood of an adverse outcome to said subject, wherein a subject identified with a high likelihood of an adverse outcome is assigned for medical intervention, and wherein the method is a two-site immunoassay method that employs an immobilized anti-histone H3.1 antibody directed to bind to a core histone epitope of histone H3.1 within amino acids 29-35 of the histone H3.1 protein together with a labelled anti-nucleosome antibody directed to bind to a conformational epitope present in intact nucleosomes.

The nucleosome is the basic unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, Methods Mol. Biol. (2007) 361: 25-62).

References to "nucleosome" may refer to "cell free nucleosome" when detected in body fluid samples. It will be appreciated that the term cell free nucleosome throughout this document is intended to include any cell free chromatin fragment that includes one or more nucleosomes.

It will be understood that the cell free nucleosome may be detected by binding to a component thereof. The term "component thereof" as used herein refers to a part of the nucleosome, *i.e.* the whole nucleosome does not need to be detected. The component of the cell free nucleosomes may be selected from the group consisting of: a histone protein (*i.e.* histone H1, H2A, H2B, H3 or H4), a histone post-translational modification, a histone variant or isoform, a protein bound to the nucleosome (*i.e.* a nucleosome-protein adduct), a DNA fragment associated with the nucleosome and/or a modified nucleotide associated with the nucleosome.

According to the invention, the component thereof is histone (isoform) H3.1.

References to "nucleosomes *per se"* refers to the total nucleosome level or concentration present in the sample, regardless of any epigenetic features the nucleosomes may or may not include. Detection of the total nucleosome level typically involves detecting a histone protein common to all nucleosomes, such as histone H4. Therefore, nucleosomes *per se* may be measured by detecting a core histone protein, such as histone H4. As described herein, histone proteins form structural units known as nucleosomes which are used to package DNA in eukaryotic cells.

Normal cell turnover in adult humans involves the creation by cell division of a huge number of cells daily and the death of a similar number, mainly by apoptosis. During the process of apoptosis chromatin is broken down into mononucleosomes and oligonucleosomes which are released from the cells. Under normal conditions the levels of circulating nucleosomes found in healthy subjects is reported to be low. Elevated levels are found in subjects with a variety of conditions including many cancers, auto-immune diseases, inflammatory conditions, stroke and myocardial infarction (Holdenreider & Stieber, Crit. Rev. Clin. Lab. Sci. (2009) 46(1): 1-24).

Previous nucleosome ELISA methods were used primarily in cell culture, usually as a method to detect apoptosis (Salgame et al., Nucleic Acids Res. (1997) 25(3): 680-681; Holdenrieder *et al.* (2001) *supra*; van Nieuwenhuijze et al., Ann. Rheum. Dis. (2003) 62: 10-14), but are also used for the measurement of circulating cell free nucleosomes in serum and plasma (Holdenrieder *et al.* (2001)). Cell free serum and plasma nucleosome levels released into the circulation by dying cells have been measured by ELISA methods in studies of a number of different cancers to evaluate their use as a potential biomarker.

The cell free nucleosome comprising histone H3.1 may be mononucleosomes, oligonucleosomes, a constituent part of a larger chromatin fragment or a constituent part of a NET or a mixture thereof.

Mononucleosomes and oligonucleosomes can be detected by Enzyme-Linked ImmunoSorbant Assay (ELISA) and several methods have been reported (*e.g.* Salgame *et al.* (1997); Holdenrieder *et al.* (2001); van Nieuwenhuijze *et al.* (2003)). These assays typically employ an anti-histone antibody (for example anti-H2B, anti-H3 or anti-H1, H2A, H2B, H3 and H4) as capture antibody and an anti-DNA or anti-H2A-H2B-DNA complex antibody as detection antibody.

Circulating nucleosomes are not a homogeneous group of protein-nucleic acid complexes. Rather, they are a heterogeneous group of chromatin fragments originating from the digestion of chromatin on cell death and include an immense variety of epigenetic structures including particular histone isoforms (or variants), post-translational histone modifications, nucleotides or modified nucleotides, and protein adducts. It will be clear to those skilled in the art that an elevation in nucleosome levels will be associated with elevations in some circulating nucleosome subsets containing particular epigenetic signals including nucleosomes comprising particular histone isoforms (or variants), comprising particular post-translational histone modifications, comprising particular nucleotides or modified nucleotides and comprising particular protein adducts. Assays for these types of chromatin fragments are known in the art (for example, see WO 2005/019826, WO 2013/030579, WO 2013/030578, WO 2013/084002).

A number of proteins occur in NETs that are adducted directly or indirectly to nucleosomes. These proteins include, without limitation, myeloperoxidase (MPO), neutrophil elastase (NE), lactotransferrin, azurocidin, cathepsin G, leukocyte proteinase 3, lysozyme C, neutrophil defensin 1, neutrophil defensin 3, myeloid cell nuclear differentiation antigen, S100 calcium-binding protein A8, S100 calcium-binding protein A9, S100 calcium-binding protein A12, actin β, actin γ, alpha-actin, plastin-2, cytokeratin-10, catalase, alpha-enolase and transketolase (Urban et al., PLOS Pathogens. (2009) 10: e1000639).

It will be understood that more than one epigenetic feature of cell free nucleosomes may be detected in methods of the invention. Multiple biomarkers may be used as a combined biomarker. Therefore, in one embodiment, the method comprises measuring more than one epigenetic feature of cell free nucleosomes as a combined biomarker. In one embodiment, the combined biomarker is H3.1 and H3cit. In an alternative embodiment, the combined biomarker is H3.1 and H4cit.

It will be understood that the terms "epigenetic signal structure" and "epigenetic feature" are used interchangeably herein. They refer to particular features of the nucleosome that may be detected. In one embodiment, the epigenetic feature of the nucleosome measured as a combined biomarker is selected from the group consisting of: a post-translational histone modification, a histone isoform, a modified nucleotide and/or proteins bound to a nucleosome in a nucleosome-protein adduct.

The term "histone variant" and "histone isoform" may be used interchangeably herein. The structure of the nucleosome can also vary by the inclusion of alternative histone isoforms or variants which are different gene or splice products and have different amino acid sequences. Many histone isoforms are known in the art. Histone variants can be classed into a number of families which are subdivided into individual types. The nucleotide sequences of a large number of histone variants are known and publicly available for example in the National Human Genome Research Institute NHGRI Histone Database (Mariño-Ramírez et al. The Histone Database: an integrated resource for histones and histone fold-containing proteins. Database Vol.2011. and http://genome.nhgri.nih.gov/histones/complete.shtml), the GenBank (NIH genetic sequence) Database, the EMBL Nucleotide Sequence Database and the DNA Data Bank of Japan (DDBJ). For example, variants of histone H2 include H2A1, H2A2, mH2A1, mH2A2, H2AX and H2AZ. In another example, histone isoforms of H3 include H3.1, H3.2, H3.3 and H3t.

According to the invention, the histone isoform is H3.1 is detected.

The structure of nucleosomes can vary by post translational modification (PTM) of histone proteins. PTM of histone proteins typically occurs on the tails of the core histones and common modifications include acetylation, methylation or ubiquitination of lysine residues as well as methylation or citrullination of arginine residues and phosphorylation of serine residues and many others. Many histone modifications are known in the art and the number is increasing as new modifications are identified (Zhao and Garcia (2015) Cold Spring Harb Perspect Biol, 7: a025064). Therefore, in one embodiment, the epigenetic feature of the cell free nucleosome measured as a combined biomarker may be a histone post translational modification (PTM). The histone PTM may be a histone PTM of a core nucleosome, *e.g*. H3, H2A, H2B or H4, in particular H3, H2A or H2B. In particular, the histone PTM is a histone H3 PTM. Examples of such PTMs are described in WO 2005/019826.

For example, the post translational modification may include acetylation, methylation, which may be mono-, di-or tri-methylation, phosphorylation, ribosylation, citrullination, ubiquitination, hydroxylation, glycosylation, nitrosylation, glutamination and/or isomerisation (see Ausio (2001) Biochem Cell Bio 79: 693). In one embodiment, the histone PTM is selected from citrullination or ribosylation. In a further embodiment, the histone PTM is H3 citrulline (H3cit) or H4 citrulline (H4cit). In a yet further embodiment, the histone PTM is H3cit.

In one embodiment, the histone PTM is ribosylation, also referred to as ADP-ribosylation. Post-translational histone ADP-ribosylation of nucleosomes occupying promoters of inflammatory response markers in macrophages is stimulated by exposure to lipopolysaccharides leading to elevated transcription and may have antiviral properties. Moreover, all members of the Coronavirus family contain a highly conserved macrodomain within non-structural protein 3 (nsp3) that regulates post-translational ADP-ribosylation by enzymatic removal of covalently attached ADP-ribose from protein targets. Recombinant severe acute respiratory syndrome coronavirus (SARS-CoV) strains containing mutated macrodomains with reduced nsp3 de-ADP-ribosylation activity, are less infective and elicit an early, enhanced interferon (IFN), interferon-stimulated gene (ISG), and proinflammatory cytokine response. Therefore, altered levels of circulating ADP-ribosylated nucleosomes released from macrophages are expected to be useful in methods of the invention.

Examples of such antibodies directed to bind to a group of histone modifications would include, for illustrative purposes without limitation, anti-pan-acetylation antibodies (*e.g*. a Pan-acetyl H4 antibody [H4panAc]), anti-citrullination antibodies or anti-ubiquitin antibodies.

In one embodiment, the epigenetic feature of the nucleosome measured as a combined biomarker comprises one or more DNA modifications. In addition to the epigenetic signalling mediated by nucleosome histone isoform and PTM composition, nucleosomes also differ in their nucleotide and modified nucleotide composition. Some nucleosomes may comprise more 5-methylcytosine residues (or 5-hydroxymethylcytosine residues or other nucleotides or modified nucleotides) than other nucleosomes. In one embodiment, the DNA modification is selected from 5-methylcytosine or 5-hydroxymethylcytosine.

In one embodiment, the epigenetic feature of the nucleosome measured as a combined biomarker comprises one or more protein-nucleosome adducts or complexes. A further type of circulating nucleosome subset is nucleosome protein adducts. It has been known for many years that chromatin comprises a large number of non-histone proteins bound to its constituent DNA and/or histones. These chromatin associated proteins are of a wide variety of types and have a variety of functions including transcription factors, transcription enhancement factors, transcription repression factors, histone modifying enzymes, DNA damage repair proteins and many more. These chromatin fragments including nucleosomes and other non-histone chromatin proteins or DNA and other non-histone chromatin proteins are described in the art.

In one embodiment, the protein adducted to the nucleosome (and which therefore may be used as a biomarker) is selected from: a transcription factor, a High Mobility Group Protein or chromatin modifying enzyme. References to "transcription factor" refer to proteins that bind to DNA and regulate gene expression by promoting (*i.e.* activators) or suppressing (*i.e.* repressors) transcription. Transcription factors contain one or more DNA-binding domains (DBDs), which attach to specific sequences of DNA adjacent to the genes that they regulate.

The term "biomarker" means a distinctive biological or biologically derived indicator of a process, event, or condition. Biomarkers can be used in methods of diagnosis, e.g. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

Biomarkers are also useful as companion diagnostic products for the selection of patients suitable for treatment by a particular therapy. We have demonstrated herein that tests for circulating nucleosome levels, or levels of nucleosomes containing particular epigenetic signals or structures, are useful companion products to therapies for NETs or NETosis related diseases.

The methods of the invention are directed to assigning the patient with a risk of an adverse outcome. Adverse outcomes include mortality and/or an acute event requiring immediate medical care, for example, hospitalisation (*i.e.* hospital treatment) and/or surgery. For many patients, infections are overcome by their own immune system without requiring medical intervention. However, in a number of patients, infections can proceed or increase in severity without being overcome by the immune system or the patient's own immune response to an infection may lead to an adverse outcome. For example, an adverse outcome may include an acute coronary or cardiac event (such as a myocardial infarction and/or stroke), acute multi- or single-organ failure (such as renal failure, liver failure and/or heart failure), onset of a debilitating acute condition and/or an acute respiratory condition (such as pneumonia, hypoventilation/bradypnea, acute respiratory distress syndrome (ARDS) severe acute respiratory syndrome (SARS), bronchiolitis and/or bronchitis).

Assigning the patient with a risk of an adverse outcome may assign a near- or short-term risk or may assign a medium-term risk. A near- or short-term risk includes wherein the patient may develop an adverse outcome within 30 days, such as within 2 weeks or 14 days, within 1 week or 7 days or within 5 days or less of presentation of symptoms or of a positive diagnosis. Such near- or short-term risk may also include wherein the patient may develop an adverse outcome within 30 days, such as within 2 weeks or 14 days, within 1 week or 7 days or within 5 days or less of performing the methods described herein. An example of a short term risk includes the development of NETs related complications to a COVID infection requiring hospital treatment. A medium-term risk includes wherein the patient may develop an adverse outcome more than 30 days after presentation of symptoms, a positive diagnosis and/or the performing of methods as described herein. An example of a medium term risk includes the development of so called long-COVID wherein the effects of a COVID infection may continue for many months. Thus, in one embodiment, the methods described herein assign a patient or subject with a risk of developing an adverse outcome within 2 weeks, or 14 days, of presentation of symptoms or a positive diagnosis. In a further embodiment, the methods described herein assign a risk of developing an adverse outcome within 1 week, or 7 days, of presentation of symptoms or a positive diagnosis. In a yet further embodiment, the methods described herein assign a risk of developing an adverse outcome within 5 days of presentation of symptoms or a positive diagnosis.

It will be understood that methods of the invention may also be used to identify patients who do not require hospital treatment, i.e. using the level of cell free nucleosomes comprising histone H3.1 detected to determine if the subject should not be admitted to hospital for treatment. This mode of the invention would help to identify patients who can be discharged early if they have already been admitted to hospital.

Methods and uses described herein may be tested in body fluid samples, in particular blood, serum or plasma samples. Preferably, plasma samples are used. Plasma samples may be collected in collection tubes containing one or more anticoagulants such as ethylenediamine tetraacetic acid (EDTA), heparin, or sodium citrate, in particular EDTA.

### Infections

Infections can be caused by different pathogens and environmental factors. Bacterial infections may include mycobacterial, pneumococcal and influenzae infections, such as infections (*e.g*. pneumonia) caused by Streptococcus pneumoniae, Escherichia coli, Mycobacterium tuberculosis, Haemophilus influenzae and Staphylococcus aureus. Viral infections may include infections caused by respiratory syncytial virus (RSV), influenza type A, influenza type B and coronaviruses (*e.g*. COVID-19).

The infection can be defined by the tissue affected by the disease. For example, the disease may affect the heart, brain, kidneys, liver, pancreas, lungs and/or blood and the infection may be a bacterial, viral, fungal or microbial infection known to commonly affect such tissues or organs.

Other tissues which may be affected by the disease include peripheral tissues such as limbs, hands and feet and the infection may be a bacterial infection (*e.g*. gangrene). In one embodiment, the infection and/or disease may affect multiple tissues or organs simultaneously. For example, the infection may be a bacterial infection of a limb, hand or foot and the disease may also affect the blood (*e.g*. sepsis). According to the invention, the infection is sepsis.

In one embodiment, circulating nucleosome levels comprising histone H3.1 are measured in a sample taken from a subject suffering from sepsis or septic shock, in particular to assess the prognosis of the disease. Further measurements on multiple samples taken at intervals from a subject suffering from sepsis or septic shock may be made to monitor the progress of the disease and/or to assess the efficacy of treatment.

### Diagnosis and monitoring methods

According to the present invention, there is provided a method of monitoring the progress of sepsis or septic shock in a subject, comprising:
(i) contacting a body fluid sample obtained from the subject with a binding agent to detect or measure the level of cell free nucleosomes comprising histone H3.1;
(ii) repeating step (i) on one or more occasions; and
(iii) using any changes in the level of cell free nucleosomes comprising histone H3.1to monitor the progression of the sepsis or septic shock in the subject,
wherein the method is a two-site immunoassay method that employs an immobilized anti-histone H3.1 antibody directed to bind to a core histone epitope of histone H3.1 within amino acids 29-35 of the histone H3.1 protein together with a labelled anti-nucleosome antibody directed to bind to a conformational epitope present in intact nucleosomes.

Detecting and/or quantifying may be performed directly on the purified or enriched nucleosome sample, or indirectly on an extract therefrom, or on a dilution thereof. Quantifying the amount of the biomarker present in a sample may include determining the concentration of the biomarker present in the sample. Methods of monitoring according to the invention described herein are useful to monitor development of the disease by assessing onset and progression, or to assess amelioration or regression of the disease. Methods of monitoring are also useful in methods for assessment of clinical screening, prognosis, choice of therapy, evaluation of therapeutic benefit, *i.e.* for drug screening and drug development.

The inventors herein used a 2-site immunoassay for H3.1-nucleosomes employing an immobilized anti-histone H3.1 antibody directed to bind to an epitope around amino acids 30-33 of the histone H3.1 protein to capture clipped and non-clipped nucleosomes, together with a labelled anti-nucleosome antibody directed to bind to a epitope present in intact nucleosomes but not present on isolated (free) histone or DNA nucleosome components. This type of epitope may be referred to as a "conformational nucleosome epitope" herein because it requires the native three-dimensional configuration of the target nucleosome to be intact.

H3R8Cit nucleosome measurements described herein were performed using a 2-site immunoassay employing an immobilized antibody directed to bind to nucleosomes citrullinated at arginine 8 of histone H3, together with the same labelled anti-nucleosome antibody directed to bind to a conformational nucleosome epitope.

It will be clear to those skilled in the art that the terms "antibody", "binder" or "ligand" as used herein are not limiting but are intended to include any binder capable of binding to particular molecules or entities and that any suitable binder can be used in the method of the invention. It will also be clear that the term "nucleosomes" is intended to include mononucleosomes, oligonucleosomes, NETs and any protein-DNA chromatin fragments that can be analysed in fluid media.

Methods of detecting biomarkers are known in the art. The antibody can be a monoclonal antibody or a fragment thereof. It will be understood that if an antibody fragment is used then it retains the ability to bind the biomarker so that the biomarker may be detected. A ligand/binder may be labelled with a detectable marker, such as a luminescent, fluorescent, enzyme or radioactive marker; alternatively or additionally a ligand may be labelled with an affinity tag, *e.g.* a biotin, avidin, streptavidin or His (*e.g.* hexa-His) tag. Alternatively, ligand binding may be determined using a label-free technology for example that of ForteBio Inc.

Methods of the invention may involve normalisation of marker levels. For example, the level of cell free nucleosomes containing a particular epigenetic feature may be normalised against the level of nucleosomes per se (or some other type of nucleosomes or parameter) to express the level as a proportion of nucleosomes containing the feature. For example, to express the level of citrullinated nucleosomes as the proportion of nucleosomes that are citrullinated.

In one embodiment, the method described herein is repeated on multiple occasions. This embodiment provides the advantage of allowing the detection results to be monitored over a time period. Such an arrangement will provide the benefit of monitoring or assessing the efficacy of treatment of a disease state. Such monitoring methods of the invention can be used to monitor onset, progression, stabilisation, amelioration, relapse and/or remission.

In monitoring methods, test samples may be taken on two or more occasions. The method may further comprise comparing the level of the biomarker(s) present in the test sample with one or more control(s) and/or with one or more previous test sample(s) taken earlier from the same test subject, *e.g*. prior to commencement of therapy, and/or from the same test subject at an earlier stage of therapy. The method may comprise detecting a change in the nature or amount of the biomarker(s) in test samples taken on different occasions.

A change in the level of the biomarker in the test sample relative to the level in a previous test sample taken earlier from the same test subject may be indicative of a beneficial effect, *e.g*. stabilisation or improvement, of said therapy on the disorder or suspected disorder. Furthermore, once treatment has been completed, the method of the invention may be periodically repeated in order to monitor for the recurrence of a disease.

In a further embodiment the monitoring of more rapid changes due to fast acting therapies may be conducted at shorter intervals of hours or days.

Immunoassays include 2-site immunoassays or immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays as well as single-site immunoassays, reagent limited immunoassays, competitive immunoassay methods including labelled antigen and labelled antibody single antibody immunoassay methods with a variety of label types including radioactive, enzyme, fluorescent, time-resolved fluorescent and particulate labels. All of said immunoassay methods are well known in the art, see for example Salgame *et al.* (1997) and van Nieuwenhuijze *et al.* (2003).

Identifying, detecting and/or quantifying can be performed by any method suitable to identify the presence and/or amount of a specific protein in a biological sample from a subject or a purification or extract of a biological sample or a dilution thereof. In particular, quantifying may be performed by measuring the concentration of the target in the sample or samples. Biological samples that may be tested in a method of the invention include those as defined hereinbefore. The samples can be prepared, for example where appropriate diluted or concentrated, and stored in the usual manner. The present invention finds particular use in plasma samples which may be obtained from the subject.

Methods involving detection and/or quantification of one or more biomarkers of the invention can be performed on bench-top instruments, or can be incorporated onto disposable, diagnostic or monitoring platforms that can be used in a non-laboratory environment, *e.g*. in the physician's office or at the subject's bedside. In one embodiment the (near patient) immunoassay method comprises a point-of-care immunoassay instrument (e.g. the Abbott i-STAT or the LightDeck Diagnostics point-of-care immunoassay instrument).

The identification of biomarkers for a disease state permits integration of diagnostic procedures and therapeutic regimes. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus, by monitoring a biomarker of the invention, subject care can be tailored precisely to match the needs determined by the disorder and the pharmacological profile of the subject, the biomarker can thus be used to titrate the optimal dose, predict a positive therapeutic response and identify those subjects at high risk of severe side effects.

Biomarker-based tests provide a first line assessment of 'new' subjects, and provide objective measures for accurate and rapid diagnosis, not achievable using the current measures.

Biomarker monitoring methods, biosensors, point-of-care tests, lateral flow tests and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

References to "subject" or "patient" are used interchangeably herein. The subject may be a human or an animal subject. In one embodiment, the subject is a human. In one embodiment, the subject is a (non-human) animal. The panels and methods described herein may be performed *in vitro,* or *ex vivo.*

Detecting and/or quantifying may be compared to a cut-off level. Cut-off values can be predetermined by analysing results from multiple patients and controls, and determining a suitable value for classifying a subject as with or without the disease. For example, for diseases where the level of biomarker is higher in patients suffering from the disease, then if the level detected is higher than the cut-off, the patient is indicated to suffer from the disease. Alternatively, for diseases where the level of biomarker is lower in patients suffering from the disease, then if the level detected is lower than the cut-off, the patient is indicated to suffer from the disease. The advantages of using simple cut-off values include the ease with which clinicians are able to understand the test and the elimination of any need for software or other aids in the interpretation of the test results. Cut-off levels can be determined using methods in the art.

Detecting and/or quantifying may also be compared to a control. It will be clear to those skilled in the art that the control subjects may be selected on a variety of basis which may include, for example, subjects known to be free of the disease or may be subjects with a different disease (for example, for the investigation of differential diagnosis). The "control" may comprise a healthy subject, a non-diseased subject and/or a subject without an infection. The control may also be a subject with the infection displaying no, or mild, symptoms, such as a subject infected with a respiratory virus displaying no, or mild, symptoms. Mild symptoms may include manageable symptoms which do not require hospital intervention and/or intensive medical treatment.

Comparison with a control is well known in the field of diagnostics. The range of values found in the control group may be used as a normal or healthy or reference range against which the values found for test subjects can be compared. For example, if the reference range is <10 units, then a test value of 5 units would be considered normal, or not in need of treatment, but a value of 11 units would be considered abnormal and indicative of a need for treatment.

Therefore, in one embodiment, the method additionally comprises comparing the level of cell free nucleosomes comprising histone H3.1in the body fluid sample of the subject with one or more controls. For example, the method may comprise comparing the level of cell free nucleosomes comprising histone H3.1 present in a sample obtained from the subject with the level of cell free nucleosomes comprising histone H3.1 present in a sample obtained from a normal subject. The control may be a healthy subject.

In one embodiment, the level of cell free nucleosomes comprising histone H3.1 is elevated compared to the control.

It will be understood that it is not necessary to measure control levels for comparative purposes on every occasion. For example, for healthy/non-diseased controls, once the 'normal range' is established it can be used as a benchmark for all subsequent tests. A normal range can be established by obtaining samples from multiple control subjects without an infection and testing for the level of biomarker. Results (*i.e.* biomarker levels) for subjects suspected to have an infection can then be examined to see if they fall within, or outside of, the respective normal range. Use of a 'normal range' is standard practice for the detection of disease.

In one embodiment, the method additionally comprises determining at least one clinical parameter for the patient. This parameter can be used in the interpretation of results. Clinical parameters may include any relevant clinical information for example, without limitation, body temperature, gender, weight, Body Mass Index (BMI), smoking status and dietary habits. Therefore, in one embodiment, the clinical parameter is selected from the group consisting of: body temperature, age, sex and body mass index (BMI).

### Additional biomarkers

The level of cell free nucleosomes may be detected or measured as one of a panel of measurements. The panel may comprise different epigenetic features of the nucleosome as described hereinbefore (*e.g*. a histone isoform and a PTM). Biomarkers useful in a panel test for the detection of severe respiratory infections that require medical intervention include, without limitation, cytokine moieties (particularly interleukins), C-reactive protein, myeloperoxidase, D-Dimer, factor Vll-activating protease (FSAP), fibrinogen and fibrin/fibrinogen breakdown products. In one embodiment, the panel comprises C-reactive protein. In one embodiment, the panel comprises one or more cytokines, such as one or more interleukins.

Interleukins (ILs) are a group of cytokines, usually secreted by leukocytes, that act as signal molecules. They have key roles in stimulating immune responses and inflammation. They were first identified in the 1970s and have been designated numerically as more interleukin types have been discovered. Examples of interleukins include, but are not limited to: IL-1, IL-2, IL-3, IL-4, IL-5, IL-6, IL-7, IL-8, IL-9, IL-10, IL-11, IL-12, IL-13, IL-14 and IL-15.

In one embodiment, the one or more interleukins is selected from the group consisting of: Interleukin-6 (IL-6) and Interleukin-12 (IL-12).

The interleukin may be IL-6. Interleukin-6 (IL-6) is a cytokine with a wide variety of biological functions. It is a potent inducer of fever and the acute phase response. The sequence of human IL-6 is known in the art and is described at UniProt Accession No. P05231. In one particular embodiment, the interleukin may be IL-6.

Alternatively, or additionally, the interleukin may be IL-12. Interleukin-12 (IL-12) is a T cell stimulating factor because it stimulates the growth and function of T cells. It is a heterodimeric cytokine comprised of IL-12A and IL-12B. The sequence of human IL-12A is known in the art and is described at UniProt Accession No. P29459 and the sequence of human IL-12B is also known and described at UniProt Accession No. P29460. In one particular embodiment, the interleukin may be IL-12.

For example, the cell free nucleosome measurement can be combined with more than one interleukin measurement, such as IL-6 and IL-12. In one embodiment, the panel of measurements is H3.1, H3cit, H4cit and IL-6.

In one embodiment, the panel comprises C-reactive protein (CRP). CRP is a pentameric protein found in plasma and levels of CRP (whether or not adducted to nucleosomes) increase in plasma in response to inflammation, such as in bacterial, viral, fungal and microbial infections. CRP levels increase following IL-6 secretion by macrophages and T cells and its physiological role is to bind lysophosphatidylcholine expressed on the surface of dead or dying cells in order to activate the complement system via C1q. It also binds to phosphocholine on the surface of some bacteria and enhances phagocytosis. The measurement of CRP levels is useful for determining the progression of disease and the effectiveness of treatments and elevated CRP levels have been shown in patients with increased risk of diabetes, hypertension and cardiovascular disease. Increased CRP levels have also been found in patients with kidney failure and inflammatory bowel disease (IBD, including Crohn's disease and ulcerative colitis) and roughly correlate with coronary heart disease, although as elevated CRP is not directly related to heart disease it is not a specific prognostic marker. Since CRP is increased during inflammation, viral infections, such as SARS or coronavirus (*e.g*. COVID-19) may also lead to increased CRP levels in plasma.

In one embodiment, the panel comprises myeloperoxidase (MPO). MPO is expressed in neutrophil granulocytes and produces hypohalous acids to carry out their antimicrobial activity. It is stored in azurophilic granules and released into the extracellular space during degranulation. The levels of MPO have been shown to be a useful predictor for myocardial infarction and have been combined with measurement of CRP for increased accuracy in predicting myocardial infarction risk in patients. In one embodiment, the panel comprises neutrophil elastase (NE).

Models can be derived using the biomarkers of the invention. Methods for deriving models or algorithms are well known in the art and suitable software packages are available. Typical software tools for this purpose include SPSS (Statistical Package for the Social Sciences) and "R". These software packages provide for linear and non-linear data modelling of clinical data.

It will be clear to those skilled in the art, that any combination of the biomarkers disclosed herein may be used in panels and algorithms for the detection or prediction of a complication to an infection, and that further markers may be added to a panel including these markers.

### Immunoassays

NETs or NETosis related diseases include infectious diseases such as sepsis, pneumonia, COVID and influenza as well as other diseases involving a pathological elevation in NETs production including, without limitation, pneumonia, SARS or ARDS of any cause, thrombotic or micro-thrombotic conditions, many inflammatory disease conditions and NETosis related complications of other diseases including amputation and thrombotic complications of diabetes and thrombotic complications of cancer and many other diseases.

Prevention or inhibition of NETosis through treatment with NETosis inhibitors reduces the level of NETs and nucleosomes in the circulation and/or tissues of subjects suffering from conditions involving inappropriately high levels of NETs. This has been shown to lead to improved clinical outcomes for subjects suffering from NETosis related disease conditions such as sepsis or stroke (Figueiredo et al., Immunity (2013) 39: 874-884 and Zhang et al., Science Advances (2019) 5: eaax7964). Similarly, removal of NETs and nucleosomes from the circulation and/or tissues of a subject suffering from NETosis associated disease conditions leads to improved clinical outcomes.

The immunoassays described herein for the measurement of H3.1-nucleosomes, citrullinated nucleosomes, MPO and NE use high avidity and specificity monoclonal antibodies for binding to nucleosomes and NETs. These antibodies bind strongly and specifically to NETs, NETs metabolites and nucleosomes. The anti-histone H3.1 antibody, anti-nucleosome antibody and anti-citrullinated H3 antibody used by the inventors for the assay of nucleosomes described herein, have been selected as highly avid and specific antibodies.

In particular, the anti-histone H3.1 antibody may be highly specific. Nucleosomes are subject to clipping in which the histone tail is physically and irreversibly removed by regulated proteolysis, or clipping. Furthermore, histone degradation has been shown to be involved in the formation of NETs (see Papayannopoulos et al. (2010) J. Cell Biol. 191(3): 677-691). On histone H3, clipping is reported to occur around amino acid position 21 (Yi and Kim (2018) BMB Reports, 51(5): 211-218). The amino acid sequence of histone H3.1 at positions 27-36 is KSAPATGGVK (SEQ ID NO: 1). The amino acid sequence at positions 29-35 does not include any commonly post-translationally modified amino acids (for example lysine, serine or arginine). Therefore, antibodies directed to bind to this epitope (i.e. amino acid positions 29-35) are unaffected, or minimally affected, by the post-translational modification status of the nucleosome, and will bind to all or most nucleosomes containing histone H3.1, regardless of PTM structure. Therefore, the solid phase capture antibody selected for use by the inventors for the immunoassay described herein, was an anti-histone H3.1 antibody directed to bind to an epitope located within the core of the histone near to amino acid position 30-33 so that both intact and clipped nucleosomes are captured by the antibody regardless of their PTM status. This maximises the capture of H3.1-nucleosomes and the efficacy of this approach is clear in Figure 3. The amino acid sequence of histone H3.1 is known in the art and is described at UniProt Accession No. P68431.

In a preferred embodiment, the anti-histone H3.1 antibody is directed to bind to an epitope located within the core of the histone H3, at or near to amino acid position 29-35, in particular at or near to amino acid position 30-33.

The labelled antibody used by the inventors herein for immunoassay was an anti-nucleosome antibody directed to bind to a conformational nucleosome epitope present in intact nucleosomes containing a histone octamer core complexed with DNA. The antibody does not bind (or binds weakly) to free histone octamer complexes, free histones (i.e. without DNA), free DNA or free histones. Again, the antibody may be relatively unaffected by the histone PTM composition of the nucleosomes to be bound.

Therefore, in one embodiment of the invention the anti-nucleosome antibody is directed to bind to a conformational nucleosome epitope present in intact nucleosomes containing a histone octamer core complexed with DNA.

It will be understood that the embodiments described herein may be applied to all aspects of the invention, *i.e.* the embodiment described for the uses may equally apply to the claimed methods and so forth.

The invention will now be illustrated with reference to the following non-limiting examples.

### EXAMPLE 1

We induced NETs formation in white cells in fresh healthy whole blood samples by addition of heparin and then demonstrated detection of the NETs material produced in plasma (Lelliott et al, International Immunology (2019) pii: dxz084). We collected whole blood samples from two healthy volunteers, in whom low levels of circulating NET material would be expected, in EDTA plasma blood collection tubes and in heparin plasma blood collection tubes. The two EDTA plasma blood collection tubes were centrifuged immediately to separate the cellular and plasma fractions to minimise contamination by large chromatin (including NET material) and the plasma was transferred to cryotubes and frozen. The two heparin plasma blood collection tubes were incubated at room temperature for 1 hour with gentle rotation of the tubes. The tubes were then centrifuged and the plasma was transferred to cryotubes and frozen.

The samples were assayed for nucleosomes containing histone isoform H3.1 (H3.1-nucleosomes) using an ELISA procedure in duplicate. In brief, 20µl of sample were added to a microtiter well containing magnetic particles precoated with an anti-histone H3.1 antibody. The sample was incubated and the magnetic particles were isolated and washed. An anti-nucleosome antibody directed to bind to a conformational nucleosome epitope conjugated to horse radish peroxidase was added to the magnetic particles. The particles were incubated and then isolated and washed. The bound anti-nucleosome antibody was measured using a coloured substrate reaction. The results are shown in Figure 1 and show that the NET material level was high in the heparin tubes but low in the EDTA tubes. This clearly shows that elevated levels of circulating NET material can be detected in a simple low cost immunoassay test.

### EXAMPLE 2

DNA was extracted from the two heparin and plasma samples described in EXAMPLE 1. and applied to a chip-based capillary electrophoresis instrument (Agilent Bioanalyzer) to analyse the DNA by fragment size. DNA fragments of approximately 150bp size corresponding to mononucleosomes have a retention time of approximately 60 seconds. As shown in Figure 2, the level of mononucleosome associated DNA observed was low (as expected for healthy volunteers). The DNA fragment size corresponding to NET material has a longer retention time of approximately 110 seconds. As shown in Figure 2 the level of NET material was low in EDTA plasma (as expected for healthy volunteers), but high in the heparin plasma tubes in which NET formation was stimulated by exposure to heparin. This result confirms that the elevated nucleosome levels observed in heparin plasma in EXAMPLE 1. above were NET derived and not mononucleosomes.

### EXAMPLE 3

Sepsis was induced in 16 pigs by infection with Escherichia coli (*E. coli*) bacteria administered by intravenous infusion over 3 hours (0-3 hours in Figures 3 and 4). The septic pigs were treated by a plasmapheresis method to remove NETs from the blood stream as described in WO2019053243. Briefly, whole blood was removed from the body of the pig through a tube into a plasmapheresis device, the whole blood was separated into a cell fraction and a plasma fraction, the plasma was passed through a plasmapheresis cartridge containing a binder of NETs to remove NETs from the plasma, the plasma was then re-joined with the blood cells and returned to the body of the pig. The plasmapheresis treatment was performed over 5 hours (2-7 hours in Figures 3 and 4). The plasmapheresis cartridges used for 9 of the pigs contained the NETs binder (treated pigs) and the cartridges used for the other 7 pigs contained no binder (control pigs).

Eight plasma samples were collected hourly at time points 0-7 hours post commencement of infection from each pig. Circulating nucleosomes were measured to ascertain whether the method of the invention was (i) effective as a monitor for the course of the infection and (ii) effective as a monitor for efficacy of the treatment.

In addition, plasma samples were collected from the plasmapheresis device, both upstream of the cartridge (to sample the plasma entering the NETs binder cartridge) and downstream of the cartridge (to sample the plasma leaving the NETs binder cartridge) to ascertain whether the extent of the depletion of the plasma by the NETs binder in the cartridge could be monitored by methods of the invention. Five upstream and five downstream samples were taken hourly at time points 3-7 hours post commencement of infection (3-7 hours in Figures 3 and 4).

The plasma samples were assayed for nucleosomes containing histone isoform H3.1 (H3.1-nucleosome) levels. Assay measurements for were performed by immunoassay using an automated immunoassay instrument. Briefly, calibrant or sample (50µl) was incubated with an acridinium ester labelled anti-nucleosome antibody (50µl) and assay buffer (100µl) for 1800 seconds at 37°C. Magnetic beads coated with an anti-histone H3.1 antibody (20µl) were added and the mixture was incubated a further 900 seconds. The magnetic beads were then isolated, washed 3 times and magnetic bound acridinium ester was determined by luminescence output over 7000 milliseconds.

Mean results for circulating H3.1-nucleosome levels in the control pigs and treated pigs are shown in Figure 3a. The control pigs (infected to induce sepsis but not treated) developed sepsis over the following hours and this was reflected in an observed rise in circulating H3.1-nucleosome levels. The rise in mean H3.1-nucleosome levels was clear at 1 hour (post commencement of infection) and accelerated after 3 hours which is consistent with the time course of the NETosis process. The H3.1-nucleosome levels continued to rise and reached 361ng/ml at 7 hours. A similar initial rise in mean circulating H3.1-nucleosome levels was observed in the treated pigs from 0-2 hours. Initiation of plasmapheresis treatment at 2 hours resulted in a slowing of the increase in nucleosome levels and the mean level observed at 7 hours was 150ng/ml. This is considerably lower than the mean level observed in the control pigs which demonstrates the effectiveness of the plasmapheresis method and shows that the level of H3.1 nucleosomes is an effective monitor and treatment guide for the course and extent of the sepsis disease and an effective monitor for the NETosis process *in vivo.*

Mean results for plasma H3.1-nucleosome levels measured in samples taken from within the plasmapheresis device upstream from the cartridge during operation are shown in Figure 3b. These results are similar to those observed for the mean circulating H3.1-nucleosome levels measured shown in Figure 3a.

Mean results for plasma H3.1-nucleosome levels measured in samples taken from within the plasmapheresis device downstream from the cartridge during operation are shown in Figure 3c. For the control pigs, the results of Figure 3c are similar to those in Figure 3b (and 3a) showing that passing plasma through a cartridge containing no binder of NETs did not significantly affect the observed H3.1-nucleosome level. This is consistent with the expected outcome that the level of NETs in plasma was not significantly affected by passage through a cartridge containing no binder of NETs. For the treated pigs, the results of Figure 3c are all low. This is consistent with the expected outcome that passing plasma through a cartridge containing a binder of NETs resulted in the removal of most or all NETs from the plasma. Moreover, the results show that NETs binder within the cartridge was not saturated with NETs at 7 hours and continued to bind all or most of the NETs present in plasma entering the device. Measurements of the level of H3.1-nucleosomes are therefore useful to determine when the binding material within a cartridge has become saturated and is hence no longer useful as a tool for the removal of NETs and should be exchanged for a fresh cartridge.

The combined results of Figures 3b and 3c therefore show that measurements of the level of H3.1-nucleosomes are useful as a monitor and a guide for treatments for NETosis and sepsis.

The results for circulating H3.1-nucleosome levels measured in samples taken from all 16 pigs are shown individually in Figure 4a. The mean H3.1-nucleosome level observed in control pigs at 7 hours was 361ng/ml and the level was above 120ng/ml in all control pigs (range 123-743ng/ml). In contrast, the mean H3.1-nucleosome level observed in treated pigs at 7 hours was 150ng/ml and the level was below 120ng/ml in most (7 of 9) treated pigs (range 27-111ng/ml). The results demonstrate the effectiveness of the plasmapheresis treatment method. The results also show that the level of H3.1 nucleosomes is an effective monitor and treatment guide for the course and extent of sepsis disease and an effective monitor and treatment guide for excessive NETosis *in vivo.* Moreover, the results in Figure 4a show that measurements of circulating H3.1-nucleosome levels can be used to identify individuals with elevated levels of NETs as suitable candidates for treatments to reduce levels of NETs or NETosis.

The 7 control pigs had elevated nucleosome levels and also had elevated indicators of clinical stress and required more intensive medical support than the 7 treated pigs with lower nucleosome levels. Moreover, the 2 treated pigs observed to have H3.1-nucleosome levels above 120ng/ml also had elevated indicators of clinical stress and required more intensive medical support. Therefore, the method of the invention is a successful method for the monitoring of the efficacy of NETosis treatments.

Results for plasma H3.1-nucleosome levels measured in samples taken from within the plasmapheresis device upstream from the cartridge during operation are shown individually for all 16 pigs in Figure 4b. As described above for Figure 3, the results shown in Figure 4b are similar to those in Figure 4a. The mean H3.1-nucleosome level observed in control pigs at 7 hours was 368ng/ml (range 121-629ng/ml). In contrast, the H3.1-nucleosome level observed in treated pigs at 7 hours was lower with a mean result of 143ng/ml (for all treated 9 pigs). For the 7 responder pigs the range of results at 7 hours was 34-127ng/ml.

Results for plasma H3.1-nucleosome levels measured in samples taken from within the plasmapheresis device downstream from the cartridge during operation are shown individually for all 16 pigs in Figure 4c. The mean level of H3.1-nucleosomes measured for control pigs at 7 hours was 378ng/ml (range 147-617ng/ml). In contrast, the mean level of H3.1-nucleosomes observed in plasma downstream of the cartridge at 7 hours for treated pigs was 2.4ng/ml (range 0.7-6.5ng/ml) and was below 7ng/ml at all time points for all 9 treated pigs.

The combined results of Figures 4b and 4c show that measurements of the level of H3.1-nucleosomes are useful as a monitor and a guide for treatments for NETosis and sepsis.

In combination, the results shown in Figure 4 indicate that the plasmapheresis treatment regime was successful in removing NETs from the circulation of all 9 treated pigs and that 7 of the 9 pigs responded well to that treatment but 2 were non-responders. These nucleosome results correlated extremely well with the clinical observations of the pigs.

Moreover, the 7 treatment responder pigs can be clearly differentiated from treatment non-responder pigs and untreated pigs on the basis of observed nucleosome results.

### EXAMPLE 4

Plasma samples were obtained from 20 human subjects diagnosed with sepsis and 10 healthy human subjects. The plasma samples were assayed for nucleosomes containing histone isoform H3.1 (H3.1-nucleosome) levels using an automated immunoassay instrument as described in EXAMPLE 3. Elevated levels were observed in sepsis samples compared to healthy subjects, which is likely due to the effect of NETosis at various stages of this disease (Figure 5).

## Claims

1. A method of monitoring the progress of sepsis or septic shock in a subject, comprising:
(i) contacting a body fluid sample obtained from the subject with a binding agent to detect or measure the level of cell free nucleosomes comprising histone H3.1;
(ii) repeating step (i) on one or more occasions; and
(iii) using any changes in the level of cell free nucleosomes comprising histone H3.1 to monitor the progression of sepsis or septic shock in the subject,
wherein the method is a two-site immunoassay method that employs an immobilized anti-histone H3.1 antibody directed to bind to a core histone epitope of histone H3.1 within amino acids 29-35 of the histone H3.1 protein together with a labelled anti-nucleosome antibody directed to bind to a conformational epitope present in intact nucleosomes.

2. A method of assigning a risk of an adverse outcome to a subject suffering from sepsis or septic shock, comprising:
(i) contacting a body fluid sample obtained from the subject with a binding agent to detect or measure the level of cell free nucleosomes comprising histone H3.1; and
(ii) using the level of cell free nucleosomes comprising histone H3.1 detected to assign the likelihood of an adverse outcome to said subject,
wherein a subject identified with a high likelihood of an adverse outcome is assigned for medical intervention, and
wherein the method is a two-site immunoassay method that employs an immobilized anti-histone H3.1 antibody directed to bind to a core histone epitope of histone H3.1 within amino acids 29-35 of the histone H3.1 protein together with a labelled anti-nucleosome antibody directed to bind to a conformational epitope present in intact nucleosomes.

3. The method as defined in claim 1 or claim 2, wherein the body fluid sample is a blood, serum or plasma sample.

4. The method as defined in any one of claims 1 to 3, wherein more than one epigenetic feature of cell free nucleosomes is measured as a combined biomarker, optionally wherein the epigenetic feature is a histone post translational modification (PTM), such as a histone PTM of a core nucleosome, in particular a histone H3 PTM.

5. The method as defined in claim 4, wherein the combined biomarker comprises H3cit.

6. The method as defined in any one of claims 1 to 5, wherein the immobilized anti-histone H3.1 antibody is directed to bind to an epitope at amino acids 30-33 of the histone H3.1 protein.

7. The method as defined in any one of claims 1 to 6, wherein the subject is a human or an animal subject.

8. The method as defined in claim 1, additionally comprising comparing the level of cell free nucleosomes comprising histone H3.1 in the body fluid sample of the subject with one or more controls

9. The method as defined in claim 8, wherein the control is a healthy subject, and/or wherein the level of cell free nucleosomes comprising histone H3.1 is elevated compared to the control.

10. The method as defined in any one of claims 1 to 9, wherein the level of cell free nucleosomes is detected or measured as one of a panel of measurements.

11. The method as defined in claim 10, wherein the panel comprises one or more interleukins (such as IL-6 or IL-12), C reactive protein (CRP), myeloperoxidase (MPO), D-Dimer and/or factor VII-activating protease (FSAP).

## Patentansprüche

1. Verfahren zum Überwachen des Verlaufs einer Sepsis oder eines septischen Schocks bei einem Subjekt, umfassend:
(i) Kontaktieren einer von dem Subjekt erhaltenen Körperflüssigkeitsprobe mit einem Bindemittel, um den Gehalt an zellfreien Nukleosomen, umfassend Histon H3.1, zu erfassen oder zu messen;
(ii) Wiederholen von Schritt (i) bei einer oder mehreren Gelegenheiten; und
(iii) Verwenden jeglicher Veränderungen des Gehalts an zellfreien Nukleosomen, umfassend Histon H3.1, um den Verlauf einer Sepsis oder eines septischen Schocks bei dem Subjekt zu überwachen,
wobei das Verfahren ein Zwei-Stellen-Immunoassay-Verfahren ist, das einen immobilisierten Anti-Histon-H3.1-Antikörper, der ausgerichtet ist, um an ein Kernhiston-Epitop von Histon H3.1 innerhalb der Aminosäuren 29-35 des Histon-H3.1-Proteins zu binden, zusammen mit einem markierten Anti-Nukleosom-Antikörper, der ausgerichtet ist, um an ein in intakten Nukleosomen vorhandenes Konformationsepitop zu binden, verwendet.

2. Verfahren zum Zuweisen eines Risikos eines unerwünschten Ausgangs zu einem Subjekt, das an Sepsis oder septischem Schock leidet, umfassend:
(i) Kontaktieren einer von dem Subjekt erhaltenen Körperflüssigkeitsprobe mit einem Bindemittel, um den Gehalt an zellfreien Nukleosomen, umfassend Histon H3.1, zu erfassen oder zu messen; und
(ii) Verwenden des erfassten Gehalts an zellfreien Nukleosomen, umfassend Histon H3.1, um dem Subjekt die Wahrscheinlichkeit eines unerwünschten Ausgangs zuzuordnen,
wobei ein Subjekt mit einer identifizierten hohen Wahrscheinlichkeit für einen unerwünschten Ausgang einem medizinischen Eingriff zugewiesen wird und
wobei das Verfahren ein Zwei-Stellen-Immunoassay-Verfahren ist, das einen immobilisierten Anti-Histon-H3.1-Antikörper, der ausgerichtet ist, um an ein Kernhiston-Epitop von Histon H3.1 innerhalb der Aminosäuren 29-35 des Histon-H3.1-Proteins zu binden, zusammen mit einem markierten Anti-Nukleosom-Antikörper, der ausgerichtet ist, um an ein in intakten Nukleosomen vorhandenes Konformationsepitop zu binden, verwendet.

3. Verfahren nach Anspruch 1 oder 2, wobei die Körperflüssigkeitsprobe eine Blut-, Serum- oder Plasmaprobe ist.

4. Verfahren nach einem der Ansprüche 1 bis 3, wobei mehr als ein epigenetisches Merkmal zellfreier Nukleosomen als ein kombinierter Biomarker gemessen wird, wobei das epigenetische Merkmal optional eine posttranslationale Histonmodifikation (PTM), wie eine Histon-PTM eines Kernnukleosoms, insbesondere eine Histon-H3-PTM, ist.

5. Verfahren nach Anspruch 4, wobei der kombinierte Biomarker H3cit umfasst.

6. Verfahren nach einem der Ansprüche 1 bis 5, wobei der immobilisierte Anti-Histon-H3.1-Antikörper ausgerichtet ist, um an ein Epitop an den Aminosäuren 30-33 des Histon-H3.1-Proteins zu binden.

7. Verfahren nach einem der Ansprüche 1 bis 6, wobei das Subjekt ein menschliches oder ein tierisches Subjekt ist.

8. Verfahren nach Anspruch 1, zusätzlich umfassend ein Vergleichen des Gehalts an zellfreien Nukleosomen, umfassend Histon H3.1, in der Körperflüssigkeitsprobe des Subjekts mit einer oder mehreren Kontrollen.

9. Verfahren nach Anspruch 8, wobei die Kontrolle ein gesundes Subjekt ist und/oder wobei der Gehalt an zellfreien Nukleosomen, umfassend Histon H3.1, im Vergleich zu der Kontrolle erhöht ist.

10. Verfahren nach einem der Ansprüche 1 bis 9, wobei der Gehalt an zellfreien Nukleosomen als eines von einer Reihe von Messungen erfasst oder gemessen wird.

11. Verfahren nach Anspruch 10, wobei die Reihe ein oder mehrere Interleukine (wie IL-6 oder IL-12), C-reaktives Protein (CRP), Myeloperoxidase (MPO), D-Dimer und/oder Faktor-VII-aktivierende Protease (FSAP) umfasst.

## Revendications

1. Procédé de surveillance de la progression d'une sepsis ou d'un choc septique chez un sujet, comprenant :
(i) la mise en contact d'un échantillon de fluide corporel obtenu auprès du sujet avec un agent de liaison pour détecter ou mesurer le niveau de nucléosomes acellulaires comprenant l'histone H3.1 ;
(ii) la répétition de l'étape (i) à une ou plusieurs reprises ; et
(iii) l'utilisation de toute modification du niveau de nucléosomes acellulaires comprenant l'histone H3.1 pour surveiller la progression de la sepsis ou du choc septique chez le sujet,
dans lequel le procédé est un procédé d'immunoessai à deux sites qui emploie un anticorps anti-histone H3.1 immobilisé dirigé pour se lier à un épitope d'histone central de l'histone H3.1 dans les acides aminés 29 à 35 de la protéine histone H3.1 ainsi qu'un anticorps anti-nucléosome marqué dirigé pour se lier à un épitope conformationnel présent dans les nucléosomes intacts.

2. Procédé d'attribution d'un risque d'une issue défavorable à un sujet souffrant de sepsis ou de choc septique, comprenant :
(i) la mise en contact d'un échantillon de fluide corporel obtenu auprès du sujet avec un agent de liaison pour détecter ou mesurer le niveau de nucléosomes acellulaires comprenant l'histone H3.1 ; et
(ii) l'utilisation du niveau de nucléosomes acellulaires comprenant l'histone H3.1 détecté pour attribuer la probabilité d'une issue défavorable audit sujet,
dans lequel un sujet identifié comme présentant une forte probabilité d'issue défavorable est affecté à une intervention médicale, et
dans lequel le procédé est un procédé d'immunoessai à deux sites qui emploie un anticorps anti-histone H3.1 immobilisé dirigé pour se lier à un épitope d'histone central de l'histone H3.1 dans les acides aminés 29 à 35 de la protéine histone H3.1 ainsi qu'un anticorps anti-nucléosome marqué dirigé pour se lier à un épitope conformationnel présent dans les nucléosomes intacts.

3. Procédé selon la revendication 1 ou la revendication 2, dans lequel l'échantillon de fluide corporel est un échantillon de sang, de sérum ou de plasma.

4. Procédé selon l'une quelconque des revendications 1 à 3, dans lequel plus d'une caractéristique épigénétique de nucléosomes acellulaires est mesurée comme un biomarqueur combiné, éventuellement dans lequel la caractéristique épigénétique est une modification post-traductionnelle (PTM) d'histone, telle qu'une PTM d'histone d'un nucléosome central, en particulier une PTM d'histone H3.

5. Procédé selon la revendication 4, dans lequel le biomarqueur combiné comprend H3cit.

6. Procédé selon l'une quelconque des revendications 1 à 5, dans lequel l'anticorps anti-histone H3.1 immobilisé est dirigé pour se lier à un épitope aux acides aminés 30 à 33 de la protéine histone H3.1.

7. Procédé selon l'une quelconque des revendications 1 à 6, dans lequel le sujet est un sujet humain ou animal.

8. Procédé selon la revendication 1, comprenant en outre la comparaison du niveau de nucléosomes acellulaires comprenant l'histone H3.1 dans l'échantillon de fluide corporel du sujet avec un ou plusieurs témoins.

9. Procédé selon la revendication 8, dans lequel le témoin est un sujet sain et/ou dans lequel le niveau de nucléosomes acellulaires comprenant l'histone H3.1 est élevé par rapport au témoin.

10. Procédé selon l'une quelconque des revendications 1 à 9, dans lequel le niveau de nucléosomes acellulaires est détecté ou mesuré comme l'une d'un panel de mesures.

11. Procédé selon la revendication 10, dans lequel le panel comprend une ou plusieurs interleukines (telles que l'IL-6 ou l'IL-12), la protéine C-réactive (CRP), la myéloperoxydase (MPO), le D-dimère et/ou la protéase activatrice du facteur VII (FSAP).
